# EUROPEAN PATENT SPECIFICATION

(11) **EP 3 735 277 B1**
(45) Date of publication and mention of the grant of the patent: **15.11.2023**
(21) Application number: 18826015.2
(22) Date of filing: 19.12.2018
(51) Int. Cl.: A61L 27/44, A61L 27/46, A61L 27/56, C08J 5/00, C08K 3/32, C08K 9/12, C08L 81/06, C08K 3/22, C08L 71/00

(54) **MEDICAL MATERIALS AND DEVICES**
MEDIZINISCHE MATERIALIEN UND VORRICHTUNGEN
MATÉRIAUX ET DISPOSITIFS MÉDICAUX

(30) Priority: 04.01.2018 GB 201800143
(43) Date of publication of application: 11.11.2020
(73) Proprietor: Cope, Aiden, Manchester, Greater Manchester M27 0FP (GB)
(72) Inventor: Cope, Aiden, Manchester, Greater Manchester M27 0FP (GB)
(74) Representative: Mewburn Ellis LLP
(86) International application number: PCT/EP2018/085952
(87) International publication number: WO 2019/134836

(56) References cited:
- WO-A1-2012/147114
- WO-A2-2014/131375
- CN-A- 107 283 963
- CN-A- 107 469 142
- MAHDIE SAFARPOUR ET AL: "Preparation of a Novel Polyvinylidene Fluoride (PVDF) Ultrafiltration Membrane Modified with Reduced Graphene Oxide/Titanium Dioxide (TiO 2 ) Nanocomposite with Enhanced Hydrophilicity and Antifouling Properties", INDUSTRIAL & ENGINEERING CHEMISTRY RESEARCH, vol. 53, no. 34, 19 August 2014 (2014-08-19), pages 13370-13382, XP55571053, ISSN: 0888-5885, DOI: 10.1021/ie502407g
- HO SANG JUNG ET AL: "Nanoscale graphene coating on commercially pure titanium for accelerated bone regeneration", RSC ADVANCES, vol. 6, no. 32, 4 March 2016 (2016-03-04), pages 26719-26724, XP55571057, ISSN: 2046-2069, DOI: 10.1039/C6RA03905G
- YARDNAPAR PARCHAROEN ET AL: "Bacterial Stress and Osteoblast Responses on Graphene Oxide-Hydroxyapatite Electrodeposited on Titanium Dioxide Nanotube Arrays", JOURNAL OF NANOMATERIALS, vol. 2017, 30 August 2017 (2017-08-30), pages 1-12, XP55570523, US ISSN: 1687-4110, DOI: 10.1155/2017/2194614
- Y. Y. SHI ET AL: "Electrophoretic deposition of graphene oxide reinforced chitosan-hydroxyapatite nanocomposite coatings on Ti substrate", JOURNAL OF MATERIALS SCIENCE: MATERIALS IN MEDICINE, vol. 27, no. 3, 12 January 2016 (2016-01-12), XP55571158, United States ISSN: 0957-4530, DOI: 10.1007/s10856-015-5634-9
- DÍEZ-PASCUAL ANA M. ET AL: "Effect of TiO 2 nanoparticles on the performance of polyphenylsulfone biomaterial for orthopaedic implants", JOURNAL OF MATERIALS CHEMISTRY. B, vol. 2, no. 43, 4 September 2014 (2014-09-04), pages 7502-7514, XP93014416, GB ISSN: 2050-750X, DOI: 10.1039/C4TB01101E
- YAO CHEN ET AL: "Antibacterial activities of surface modified electrospun poly(vinylidene fluoride-co-hexafluoropropylene) (PVDF-HFP) fibrous membranes", APPLIED SURFACE SCIENCE, vol. 255, no. 6, 28 October 2008 (2008-10-28), pages 3854-3858, XP028738849, ISSN: 0169-4332, DOI: 10.1016/J.APSUSC.2008.10.063
- KUMAR SWETA BINOD ET AL: "Enhanced bacterial affinity of PVDF membrane: its application as improved sea water sampling tool for environmental monitoring", ENVIRONMENTAL SCIENCE AND POLLUTION RESEARCH, SPRINGER BERLIN HEIDELBERG, BERLIN/HEIDELBERG, vol. 24, no. 6, 5 January 2017 (2017-01-05), pages 5831-5840, XP036190694, ISSN: 0944-1344, DOI: 10.1007/S11356-016-8318-1 [retrieved on 2017-01-05]

## Description

### Field of the Invention

The present invention relates to medical materials and devices, and particularly, although not exclusively, to materials and devices for use in orthopaedic applications.

### Background

A medical implant is a device typically manufactured to replace, support or enhance a missing, or damaged biological structure within the human body. In the field of medical implants for orthopaedic applications, such implants include e.g. hip prostheses, knee replacement implants, intervertebral prostheses, cranial prostheses etc.

Typically, such implants must be made out of a material which is biocompatible for the intended application. Biocompatible is generally understood to mean the ability of a material to perform with an appropriate host response in a specific application [The Williams' Dictionary of Biomaterials, D.F. Williams, 1999]. For articulating implants having bearing surfaces, e.g. hip prostheses, knee replacement implants, spinal implants, spinal disc prostheses etc., the tribological properties of the implant are also important, because excessive wear of the implant material and/or production of particulate matter within a joint may shorten the lifespan of the implant, and are generally undesirable.

A wide range of materials are presently used for such medical implants, as will be known by the skilled person. For orthopaedic, spinal and craniomaxillofacial applications, a selection of known materials includes titanium and its alloys, acrylic, ultra-high-molecular-weight polyethylene (UHMWPE), polyetheretherketone (PEEK), hydroxyapatite (HAP) and others. However, each of these materials has a number of associated problems or limitations. For example, use of titanium may result in postoperative CT & MRI scan artefacts, which can render them impractical or unsuitable for use in situations where post-operative imaging may be required. UHMWPE and acrylic are non-steam sterilisable due to the risk of material degradation at the required temperatures, whilst acrylic has both a relatively low toughness and has been indicated to present a relatively high post-surgical infection rate when used in cranial prostheses. PEEK is relatively expensive, and furthermore has a relatively low toughness compared to similar materials. HAP, whilst being known to promote osseointegration in some situations, is expensive and brittle with poor malleability, and has relatively low strength resulting in risk of implant collapse *in-vivo.*

Whilst the above materials are a selection of common matrix materials, it is also known to use additional filler materials to improve various properties of the matrix materials for use as a medical implant material. For example, TiO₂ is known to be a photocatalytic material that is nontoxic, and generally chemically stable. There has been some work done on incorporating TiO₂ into materials for orthopaedic applications. E.g. Visai et al (2011) have reported on use of TiO₂ for use in surface coatings for biomedical devices. However, providing a surface coating to a device requires one or more additional manufacturing stages after formation of the device. Furthermore, care must be taken to ensure adequate attachment of the coating to the device, due to the risk of production of particular matter from the coating in-vivo which may be undesirable.

Diez-Pascual et al (2014) have reported on the properties of nanocomposites of biocompatible polyphenylsulphone and titanium dioxide nanoparticles prepared via ultrasonication and solution casting. Here, it was shown that the nanocomposites exhibited inhibition against Gram-positive and Gramnegative bacteria. However, here it was shown that loading of TiOz was not always effective for bacterial growth inhibition without UV-irradiation, particularly at low loading amounts. Accordingly, the materials proposed therein may not provide suitable antibacterial effect on implantation *in-vivo,* where UV irradiation of the implants will not occur.

CN107469142 A proposes an anti-bacterial modified nanocomposite material which is prepared from 52-85% silver carried titanium nano material, 24-56% of graphene oxide, 1-15% of nano ceramic material and 1-10% of nano apatite. The nanocomposite material disclosed therein is surface modified with PEG.

It would be desirable to provide a material which provides superior antibacterial, mechanical and/or tribological properties compared to known alternatives.

One procedure in which medical implants are commonly used is cranioplasty. Cranioplasty is a medical procedure used to repair cranial defects, and may be required for a number of indications including brain haemorrhage requiring decompressive craniectomy for reducing raised intracranial pressure, or for intracranial disorders caused by trauma, tumours or infection. Whilst it is possible to use autologous bone for cranioplasty procedures, this has a number of disadvantages. The procedure may be complex and present a higher risk of infection due to multiple surgical sites. Only a limited quantity of bone can be taken from a donor site, which may be insufficient for large and/or complex defects. Furthermore, there is a possibility of donor site morbidity, and the risk of reabsorption of the donor bone, particularly in children. So, a cranial prosthesis is commonly used instead, generally being made out of materials as discussed above. While cranial prostheses made from the above materials will clearly suffer from the associated problems, there are a number of additional problems which must be considered which are specific to craniomaxillofacial applications. Firstly, the cosmesis of craniomaxillofacial surgeries is often considered to be a highly important factor. It is desirable that the final post-surgery result looks as 'natural' as possible. For some present cranial prostheses, e.g. most present titanium sheet and mesh prostheses, it is necessary for the prosthesis to overlap the edges of the cranial defect to ensure satisfactory attachment of the prosthesis to the skull. This can reduce cosmesis.

Furthermore, another issue which sometimes occurs after cranioplasty surgery is the need for subsequent revision surgery. However, because current prostheses fix directly into, and are intended to form a bond with the existing skull, such revision surgery generally necessitates major surgical intervention and total removal of the pre-existing prosthesis, which can increase the risk of such surgery.

The present invention has been devised in light of the above considerations.

### Summary of the Invention

Proposed herein are materials and devices for use in orthopaedic applications. The term "orthopaedic applications" is used herein to refer generally to applications associated with the musculoskeletal structures and systems of the body, for example the correction or prevention of deformities, disorders, or injuries of the musculoskeletal structures and systems of the body due to degenerative, tumour, trauma, infection, developmental, congenital, metabolic, arthritic, surgical or other causes. Orthopaedic applications includes such applications as manufacture of orthopaedic, craniomaxillofacial and spinal implants or prostheses.

The present invention provides an orthopaedic implant as set out in claim 1. That is, in a first aspect, there is provided an orthopaedic implant comprising a composite material, the composite material comprising a polymeric matrix material and filler material comprising a TiO₂-reduced graphene oxide nanocomposite.

As discussed above, TiO₂ exhibits photocatalytic activity under suitable irradiation, such as by irradiation with ultraviolet (UV) radiation. In other words, TiO₂ is a photoactive material. When irradiated with radiation such as UV light, gamma rays, or any other suitable form of radiation, TiO₂ undergoes charge separation according to the following mechanism:

*TiO₂* + *hυ → TiO₂ (h* + *e)*

This charge separation can be considered as 'photoactivation' of the TiO₂. The charge carriers which are formed promote formation of reactive species and in particular reactive oxygen species such as hydroxyl and peroxide radicals and superoxide anions at the material surface. Such reactive species are theorised to cause damage to bacteria cell membranes. Provision of a filler material comprising TiO₂ therefore allows for a photoactive antibacterial effect which may be beneficial in orthopaedic applications.

Advantageously, by providing a filler material which further comprises a suitable electron-accepting or charge-scavenging material in addition to TiO₂, electrons formed upon irradiation such as UV-, gamma-, or electron beam irradiation of TiO₂ particles can be transferred to the electron accepting material. This charge transfer may prevent or reduce subsequent recombination of the electrons and holes produced upon irradiation. It is theorised that this reduced recombination of charge carries promotes the formation of reactive species at the surface of the material, thus improving antibacterial properties. Furthermore, it is also suggested that the reduced recombination may allow for continued formation of reactive species even in the absence of suitable illumination, making the material particularly suitable for use in applications where UV illumination, or irradiation by other suitable means (e.g. gamma irradiation, or electron beam irradiation) is not possible or readily practicable, such as in *in vivo* implantation of the material. Finally, another advantage of providing this 'active' material as a filler material in a composite rather than e.g. as a surface coating includes improved ease of manufacture and reduced risk of particulate formation in use, particularly when used for making e.g. articulating orthopaedic implants.

Whilst it is hypothesised that any suitable electron-accepting or charge-scavenging material may be suitable for achieving such an effect, the present inventors propose that graphene (GR), graphene oxide (GO) and/or reduced graphene oxide (rGO) may be particularly suitable for use in materials for orthopaedic applications, because of their known biocompatibility and other desirable properties that will be discussed further below, including tribological properties. For example such materials, in particular graphene, may act as solid lubricants within the polymeric matrix material to improve the tribological properties of the composite material, and in particular may improve the wear resistance of the composite material. The material selected from graphene (GR), graphene oxide (GO) and/or reduced graphene oxide (rGO) may be in the form of nanoplatelets. It is also theorised that other carbon nanostructures such as carbon nanotubes and fullerene structures may be suitable electron-accepting or charge-scavenging materials. However, the present invention employs a TiOz-reduced graphene oxide nanocomposite.

A further property of TiO₂ includes radiation activated (such as UV-activated, gamma-activated or electron beam-activated) superhydrophilicity. This effect is a result of a similar mechanism as discussed above in relation to antibacterial effect of TiO₂, i.e. due to photogeneration of electron hole pairs upon irradiation of TiO₂ with e.g. UV light or gamma rays (photoactivation). A superhydrophilic surface is generally defined as a surface having a very low contact angle, for example a zero- or almost-zero degree contact angle for water droplets. The exact mechanism by which TiO₂ becomes a superhydrophilic surface as a result of irradiation is not precisely known, but it is theorised that photogenerated holes in TiO₂ migrate to the surface and weaken the bond between titanium and oxygen atoms, the oxygen atoms being liberated to create oxygen vacancies with the conversion of Ti⁴⁺ to Ti³⁺. It may then be possible for water molecules to become chemisorbed at these Ti³⁺ sites, thus creating a hydroxylated surface with increased hydrogen bonding activity. Increased hydrogen bonding activity may accordingly provide a superhydrophilic effect. In a similar manner as that discussed above, by providing a filler material comprising a combination of TiO₂ and a suitable electron-accepting or charge-scavenging material such as graphene, graphene oxide, or reduced graphene oxide, the superhydrophilic effect may be synergistically and advantageously enhanced due to reduced recombination of electron-hole pairs. Furthermore, it is also suggested that this reduced recombination may allow for continued superhydrophilicity even in the absence of suitable illumination, making the material particularly suitable for use in applications where UV illumination, or irradiation by other suitable radiation (e.g. gamma rays) is not possible or readily practicable, such as in *in vivo* implantation of the material.

A filler material comprising TiO₂ and a material selected from graphene, graphene oxide or reduced graphene oxide could in theory be used to form a composite material by combination with a number of different matrix materials. However, in the field of materials for use in orthopaedic applications it may be particularly advantageous to use a polymeric matrix material, because such materials may allow for improved ease of manufacture compared to other known alternatives (e.g. the ability to manufacture articles from the material using 3D printing methods such as fusion deposition modelling). Furthermore, use of a polymeric matrix material can reduce or avoid CT & MRI scan artefacts.

The term 'filler material' is intended to refer generally to materials (particulate, fibrous or other) added to the polymeric matrix material for a range of purposes including lowering consumption of the matrix material and altering the properties of the matrix material. Filler material may herein refer to both a single material for use as a filler material, and also more generally to refer to a mixture of materials used as filler materials.

TiO₂ as referred to herein may include all phases of TiO₂ (anastase, rutile, brookite etc.) and combinations thereof. For example, the TiO₂ may be entirely in the anastase phase. Alternatively the TiO₂ may be e,g, 80% anastase and 20% rutile. The exact phase composition of the TiO₂ is not particularly limited and may be selected as appropriate for the particular application.

The average particle diameter of TiO₂ may be from 5 nm to 50 nm, preferably from 10 nm to 30 nm.

The reduced graphene oxide may comprise sheets or nanoplatelets. The thickness of the nanoplatelets or sheets may be from 0.25 nm to 10 nm, preferably from 0.4 nm to 2 nm. The average diameter of the nanoplatelets or sheets may be from 1 µm to 20 µm, preferably from 1.5 µm to 5.5 µm.

The amount of filler material may be from 0.05 wt% to 80 wt% based on total weight of the composite material. That is, the amount of filler material based on total weight of the composite may be 0.05wt%, 1 wt%, 2 wt%, 3 wt%, 5 wt% or 10 wt% or more, up to 80 wt%, 70 wt%, 60 wt%, 50 wt% or less. It will be understood that the total amount of filler material is not particularly limited other than to the extent that the composite material remains suitable for use in orthopaedic applications.

The amount of TiO₂ as a filler material in the composite material may be from 0.05wt %, 0.5 wt%, 1 wt%, 1.5 wt%, 2 wt%, 2.5 wt%, 3 wt%, 5 wt% or 10 wt% or more, up to 80 wt%, 70 wt%, 60 wt%, 50 wt% or less, based on total weight of the composite material. The precise amount of TiO₂ may be selected as appropriate for the particular application. Because of the synergistic effect achieved by the combination of TiO₂ and a material selected from graphene, graphene oxide or reduced graphene oxide, it is theorised that a suitable antibacterial effect can still be achieved even at low concentrations. Accordingly, the amount of TiO₂ as a filler material in the composite material may be less than 3wt%, less than 2wt% or less than 1wt% based on total weight of the composite material.

The amount of reduced graphene oxide may be from 0.05wt%, 0.5 wt%, 1 wt%, 1.5 wt%, 2 wt% or 2.5 wt% or more, up to 30 wt%, 20 wt%, 10 wt% or less, based on total weight of the composite material. The precise amount of this electron-accepting or charge-scavenging material may be selected as appropriate for the particular application. Adding greater amount of this material may improve the mechanical properties of the composite material. However, it may also be desirable to reduce or minimise the amount used due to the cost of these materials.

The combined amount of TiO₂ and the reduced graphene oxide may be less than 3 wt%, less than 2 wt% or less than 1 wt% based on total weight of the composite material. Again, it is theorised that the synergistic effect of providing a combination of these materials can reduce the need for inclusion of a large amount of these materials.

The ratio of TiO₂ to reduced graphene oxide (GR/GO/rGO) in the filler material may be 50:50, 60:40, 70:30, 80:20, 90:10, 95:5, or 99:1 by weight. In other words, the amount of TiO₂ may be from 50 wt% to 99 wt% based on combined weight of TiO₂ and GR/GO/rGO. Preferably, the ratio of TiO₂ to GR/GO/rGO is 80:20 by weight.

The filler material may further comprise hydroxyapatite (also known in the art as hydroxylapatite). Hydroxyapatite is a naturally occurring mineral form of calcium apatite with the formula Cas(PO₄)₃(OH). Hydroxyapatite in the form of bone mineral makes up approximately up to 70 wt% of human bone. Accordingly, by including hydroxyapatite as a filler material in the composite material, bone ingrowth may be promoted.

Where the composite material comprises hydroxyapatite as a filler material, the amount of hydroxyapatite may be from 1 wt% to 70 wt% based on total weight of the composite material. That is, the amount of hydroxyapatite based on total weight of the composite may be from 1 wt% or more, 2 wt% or more, 3 wt% or more, 5 wt% or more or 10 wt% or more, up to 70 wt% or less, 60 wt% or less, 50 wt% or less, 40 wt% or less, or 30% or less. Providing a higher amount of HAP may assist in promoting bone integration. However, it may be desirable for the amount of HAP to be below a threshold value so that the composite material has adequate strength.

Preferably the polymeric matrix material is selected from polyphenylsulphone, polycarbonate urethane, a PAEK (polyaryletherketone), polycaprolactone, poly -L- lactic acid, polyvinyl acetate or poly(lactic -co-glycolic acid). Where the polymeric matrix material is a PAEK, preferably it is PEEK (polyetheretherketone) or PEKK (polyetherketoneketone). The skilled person will understand that a wide range of other polymeric matrix materials may be suitable, however these polymers are particularly preferred due to their known biocompatibility and regulatory approval status. More preferably, the polymeric matrix material is a polyphenylsulphone. Polyphenylsulphone (PPSU) has superior toughness compared to some alternatives, and furthermore may offer a close property match to cranial bone in terms of features such as density, tensile strength, compressive strength and impact resistance

The filler material comprises a TiO₂-reduced graphene oxide nanocomposite. A TiO₂-reduced graphene oxide nanocomposite may be produced by any suitable method. However, methods such as UV-assisted photocatalytic reduction of graphene oxide in the presence of TiO₂, or by hydrothermal methods such as that proposed by Fan et al. (2011) may be particularly convenient. Such nanocomposite materials can have 'hybrid' material properties. For example, a TiOz-reduced graphene oxide nanocomposite material may comprise heterojunctions on the nanoscale. It is theorised that the formation of such heterojunctions can promote the efficient separation of electron-hole pairs under irradiation, discussed above, thus leading to improved photocatalytic activity.

As discussed above, such nanocomposite materials are photoactive, and can be photoactivated by irradiation with suitable radiation, for example by irradiation with UV- or Gamma- radiation, or by electronbeam irradiation (EBI). This irradiation may take place either during or after formation of the nanocomposite. If the irradiation takes places during formation of the nanocomposite material (e.g. during UV-assisted photocatalytic reduction of graphene oxide in the presence of TiO₂), the irradiation time may be a few hours or more. Preferably the irradiation time is 5 hours or more, although irradiation of up to 24 hours or more, or 48 hours or more is contemplated.

The method of photoactivation is not particularly limited. One particularly convenient method of UV-irradiating such materials may be to use a low pressure mercury lamp - in particular, lamps having narrow band UV emission at a wavelength of 254nm wavelength, and a power of e.g. 16-18W. Such lamps are generally energy efficient, and relatively easy to obtain.

Another possible method of photoactivation of photoactive materials is by use of gamma irradiation, such as the gamma irradiation used in some known commercial sterilisation methods. Kralchevska et al (2012) suggests that TiO₂ photoactivated using gamma irradiation may continue to remain photoactive even when not subject to further illumination for relatively long periods of time. In this particular study, photocatalytic properties of the photoactivated TiO₂ were found to be effectively unchanged after 45 days of storage. Without wishing to be bound by theory, the present inventors contemplate that gamma irradiation may have a significant influence on the photocatalytic activity of TiO₂, through the creation of induced defects in the crystal lattice of the TiO₂ (mostly Ti3+ sites) influencing the recombination rate of electron-hole pairs. Additionally, the present inventors contemplate that gamma irradiation may create a higher density of e-h pairs compared to lower energy irradiations such as UV or visible light. Accordingly, photoactivation by gamma irradiation may offer particular advantages for photoactivation of materials intended for in-vivo use (for example, in orthopaedic implants), as it may provide an increased photoactive lifetime in the absence of illumination/irradiation. Additionally, gamma-irradiation may be further advantageous for photoactivation of materials intended for use in in-vivo applications because both sterilisation and photoactivation of the material can occur at the same time.

Where gamma irradiation is performed, the material may be irradiated at a dose of 8kGy-40kGy (8,000 - 40,000 JKg⁻¹). A preferred dosage is about 25kGy (25,000 JKg⁻¹). The rate of dosage may be around 10kGy/hour (10,000 JKg⁻¹hour⁻¹) although this will depend on the precise irradiation setup.

It may in some cases be advantageous to perform 'top-up' irradiation/photoactivation of the material to ensure maintenance of the photoactive properties of the material. One example of a situation where it may be desirable to perform a 'top-up' irradiation step is where such a material is used as a filler material e.g. in an orthopaedic implant. Then, it may be advantageous to perform 'top-up' irradiation immediately before implantation of the implant.

As noted above, the present invention provides an orthopaedic implant made from a material as discussed above. The orthopaedic implant may be an orthopaedic prosthesis. Preferably, the orthopaedic implant is a cranial prosthesis, or an articulating implant having one or more bearing surfaces. However, the skilled person will readily understand that the material discussed above may be employed in manufacture of a wide range of orthopaedic implants as appropriate.

For non-articulating implants such as cranial implants, as discussed above, use of such material can offer superior antibacterial properties and may according reduce infecting risk on implantation. For articulating implants intended for use in predominantly water based lubricated environments (e.g. articulating knee and spinal implants), in addition to superior antibacterial properties, the superhydrophilicity of the material as discussed above may provide superior tribological properties and reduce wear and particulate formation within the joint.

Also disclosed herein is a cranial prosthesis comprising a peripheral frame portion defining an aperture, and a removable insert portion for closing the aperture.

Preferably the peripheral frame portion comprises the entire periphery of the prosthesis. The frame portion may be a regular polygonal shape. However, given the nature of cranial prostheses, preferably the frame portion is shaped to match a cranial defect of an intended recipient of the prosthesis. By providing a prosthesis with a shape matching the cranial defect, cosmesis of cranioplasty surgery using the prosthesis may be improved due to reduced or zero overlap of the prosthesis with the edges of the cranial defect. The precise dimensions of the prosthesis are not particularly limited, and may be selected as appropriate for the specific application.

The peripheral frame portion may be provided with one or more integrated fixation lugs for attachment to the cranial bone. Provision of integrated lugs may provide intraoperative ease of use by removing the requirement for provision and attachment of separate fixation plates.

The aperture defined by the frame portion is not particularly limited in size or shape, however preferably should be sized to allow suitable access through the aperture for subsequent revision surgery after an initial cranioplasty procedure. Accordingly, the aperture may span 10% or more of the total area defined by the prosthesis. Preferably, the aperture spans 20%, 30%, 40% 50%, 60%, 70%, 80% or 90% or more of the total area defined by the prosthesis. Providing a larger aperture may allow easier access through the aperture for subsequent revision surgery. However, by providing a larger aperture, the mechanical strength of the prosthesis may be reduced. So, a suitable aperture size may be selected which balances these two factors. The skilled person will appreciate that this parameter may also be dependent on the overall size and shape of the prosthesis, e.g. a smaller prosthesis will likely require a relatively larger aperture to give suitable access for revision surgery, whilst a larger prosthesis will likely require a relatively smaller aperture to provide suitable mechanical strength.

Preferably the peripheral frame portion defines a single aperture for closing with a single removable insert portion. In other words, preferably the cranial prosthesis is a two-part prosthesis. Minimising the number of parts may reduce manufacturing complexity and improve intraoperative ease of attachment of the prosthesis.

The insert is provided for closing the aperture, and it is anticipated that in general use the aperture will be closed by the insert. However, the insert is removable as discussed above, and may be removed for the purpose of performing revision surgery, or for e.g. decompressive purposes to assist in removing pressure on the brain post-implantation. The insert may therefore be removably fixed to the peripheral frame portion using e.g. screws or other suitable attachment means.

Preferably the insert portion lies flush with the peripheral frame portion when closing the aperture. Accordingly, the peripheral frame portion may have recessed fixation points for the removable insert portion such that in use, the insert portion sits flush with the frame portion. The recessed fixation points may be e.g. lugs or a recessed ledge.

The prosthesis may be formed of any material suitable for use as an orthopaedic prosthesis. As such, the prosthesis may comprise materials including but not limited to: titanium, acrylic, ultra-high-molecular-weight polyethylene (UHMWPE), polyetheretherketone (PEEK), polyetherketoneketone (PEKK), hydroxyapatite (HAP), polyphenylsulphone (PPSU), polycarbonate urethane (PCU), polycaprolactone (PCL), poly -L- lactic acid (PLLA) , polyvinyl acetate (PVA) or poly(lactic -co-glycolic acid) (PLGA). Preferably the prosthesis is formed from a composite material comprising a polymeric matrix material and a filler material. More preferably, the prosthesis is formed from a composite material as discussed above in relation to the first aspect disclosed herein.

Preferably, the peripheral frame portion of the prosthesis is porous. Preferably, the frame portion has an average porosity from 5% and up to 95%. Different regions of the frame portion may have different porosities, in a manner discussed in more detail below in relation to the fourth and fifth aspects of the invention, below. Preferably the average pore size of the peripheral frame region is from 100µm to 600µm.

In particular, the peripheral frame portion may have laterally graduated porosity. That is, the porosity of the peripheral frame portion may vary in an in-plane direction laterally across the implant. In this way the porosity of different regions of the frame portion can be selected to both encourage bone integration whilst providing suitable mechanical properties. The peripheral frame portion may have through-thickness graduated porosity. That is, the porosity of the peripheral frame portion may vary between an inner and an outer surface of the implant. In this way, the porosity of the peripheral frame portion can be selected to more closely match that found in natural cranial bone and encourage bony integration whilst providing suitable mechanical properties.

Preferably the removable insert portion has a lower porosity than the peripheral frame portion. Preferably, the average porosity of the removable insert portion is less than 5%, or less than 1%. By providing such a low porosity, bone integration into this portion of the prosthesis may be discouraged. Whilst this is against the typical teaching that bony integration should be promoted in orthopaedic implants, it is advantageous in the present invention because it may allow for ease of removal of the removable insert portion for e.g. subsequent revision surgery. Therefore more preferably, the removable insert portion is non-porous.

Where the peripheral frame portion provides fixation points (e.g. fixation lugs or a fixation ledge) for the removable insert portion, the fixation points may have a lower porosity than the rest of the peripheral frame portion. Preferably the fixation points are non-porous. In this way the fixation points may have a more suitable mechanical strength and provide for more secure fixation of the removable insert portion.

The prosthesis may be manufactured by any suitable known process including e.g. moulding, machining, or additive manufacturing. Preferably the prosthesis is manufactured using an additive manufacturing process such as e.g. fusion deposition modelling. Such process can provide a cost-effective method of producing 'custom' prostheses e.g. those which are shaped to match a cranial defect of an intended recipient of the prosthesis.

Also disclosed herein is a cranial prosthesis comprising a core layer and a first skin layer, the first skin layer having a lower porosity than the core layer. In other words, the prosthesis has a through-thickness porosity graduation thereby defining a core layer and a skin layer. A porosity graduation here also includes step-changes in porosity.

By providing a cranial prosthesis comprising multiple layers with differing porosities, it is possible to provide a prosthesis which more closely matches the morphology of cranial bone. Cranial bone typically has two main types of bone: inner trabecular bone ('spongy' or cancellous bone) having a typical porosity of around 75-85% and a typical pore size of around 300-600 µm, and outer cortical bone having a typical porosity of around 5-10% and a typical pore size of around 10-50 µm . Accordingly, a higher porosity core layer of the prosthesis may be used to match or mimic trabecular bone, whilst a lower porosity skin layer of the prosthesis may be used to match or mimic cortical bone.

Preferably, the prosthesis comprises a second skin layer disposed on the opposite side of the core layer from the first skin layer. This second skin layer may have a lower porosity than the core layer. The porosity of the second skin layer may be equal or approximately equal to the porosity of the first skin layer. In this way, the through-thickness structure may be symmetrical about the lower-density core layer.

The average porosity of the core layer may be from 50% up to 95%. Preferably, the average porosity is from 70% up to 90%. More preferably, the average porosity is from 75% up to 85%. In this way, the average porosity of the core layer may more closely match or mimic trabecular bone.

The average porosity of the skin layer(s) may be from 50% or less, 30% or less, 20% or less, 10% or less or 5% or less. Preferably the average porosity of the skin layer(s) is from 5% up to 10%. In this way, the average porosity of the core layer may more closely match or mimic cortical bone. However, the skin layer(s) may be non-porous.

The average pore size may also vary across the prosthesis. Accordingly, the average pore size of the core layer may be from 250 µm to 650 µm, more preferably from 300 µm to 600 µm. In this way, the pore size of the core layer can more closely mimic typical pore sizes found in trabecular bone. The average pore size of the skin layers may be from 50 µm to 250 µm, more preferably from 100 µm to 200 µm. In this way, the pore size of the skin layer(s) can more closely mimic typical pore sizes found in cortical bone. By more closely matching pore sizes to those found in natural cranial bone, it may be possible to provide a prosthesis which both encourages improved bone integration whilst providing suitable mechanical properties.

In typical cranial bone, the thickness of trabecular bone ranges from approximately 1.5 mm to 4 mm. The thickness of cortical bone varies from approximately 0.5 mm to 3.5 mm. Accordingly, the core layer of the prosthesis may have a thickness of 1 mm to 5mm. The skin layer(s) may (each) have a thickness from 0.25 mm to 4mm. The thickness may be selected as appropriate to more closely match that of cranial bone, whilst providing suitable mechanical strength for the prosthesis.

Preferably the cranial prosthesis is manufactured using an additive manufacturing technique or process. For example, the prosthesis may be manufactured using fusion deposition modelling. By using an additive manufacturing process it is possibly to provide a controlled pore distribution and morphology, to more closely match or mimic cranial bone than is possible using other manufacturing methods. Fusion deposition modelling may be particularly suitable due to the resolution of such processes offering the opportunity to produce parts having finer detail than in some other additive manufacture processes. E.g. the skilled person will be aware of commercially available fusion deposition modelling machines having resolutions as low as 50 µm or less, which would be suitable for manufacturing a prosthesis to the above specifications. Such fusion deposition modelling machines may have print temperatures of e.g. up to around 450 °C to allow processing of a wide range of materials. Preferably the working chamber is a closed, heated working chamber of a suitable size.

Also disclosed herein is a cranial prosthesis having laterally graduated porosity such that the porosity of a peripheral region of the implant is greater than the porosity of a central region of the implant. Where the prosthesis is a planar or plate-like prosthesis (as is typically the case for cranial prostheses), the porosity of the prosthesis may therefore vary in an in-plane direction across the prosthesis.

By having laterally graduated porosity that is greater at a peripheral region and lower at a central region, the prosthesis may encourage bone integration at the peripheral region, whilst maintaining suitable mechanical strength in the central region of the prosthesis.

A peripheral region may be a bone-contacting region of the prosthesis, or including a bone-contacting edge of the prosthesis. The peripheral region may be a region which extends inwardly from a bone-contacting edge of the prosthesis for a distance of 5cm or less, 4cm or less, 3cm or less, 2cm or less or 1 cm or less.

The average porosity of the peripheral region may be from 5% up to 95%. Preferably, the average porosity of the peripheral region is from 50% to 90%, more preferably 70% to 90%, more preferably 75% to 85%. In this way, the average porosity of the peripheral region may more closely match or mimic trabecular bone and accordingly encourage bone integration.

The average porosity of the central region may be from 10% or less, 5% or less or 1% or less. Preferably the central region is non-porous. By limiting from the porosity of the central region, or providing a non-porous central region, the mechanical strength of the central region may be improved.

The average pore size of the peripheral region may be from 50 µm to 650 µm, preferably from 100 µm to 600 µm. The average pore size of the central region may be 100 µm or less, preferably 50 µm or less. More preferably, the central region is non-porous.

The peripheral region may be defined by a frame portion of the prosthesis. The cranial prosthesis may be a cranial prosthesis having a frame portion defining an aperture and a removable insert portion for closing the aperture. Accordingly the peripheral region having higher porosity may be a frame portion defining an aperture, and the central region having lower porosity may be a removable insert portion for closing the aperture.

The invention includes the combination of the aspects and preferred features described except where such a combination is clearly impermissible or expressly avoided. In particular, as discussed above, it is contemplated that cranial prostheses discussed above may be made from the material discussed in relation to the first aspect of the invention.

### Summary of the Figures

Embodiments and experiments illustrating the principles of the invention will now be discussed with reference to the accompanying figures in which:
**Figure 1** is a schematic plan view of a cranial prosthesis according to one embodiment of the present invention;
**Figure 2** is a schematic exploded view of a cranial prosthesis according to one embodiment of the present invention;
**Figure 3** is a schematic cross-sectional view of a cranial prosthesis according to one embodiment of the present invention;
**Figure 4** is schematic cross-sectional view of a portion of a cranial prosthesis according to one embodiment of the present invention;
**Figure 5** is an oblique posterior view of a spinal prosthesis according to one embodiment of the present invention;
**Figure 6** is an oblique posterior view of a pair of members forming a part of a spinal prosthesis according to Figure 5;
**Figure 7** is an oblique anterior view of a pair of members forming a part of a spinal prosthesis according to Figure 5.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

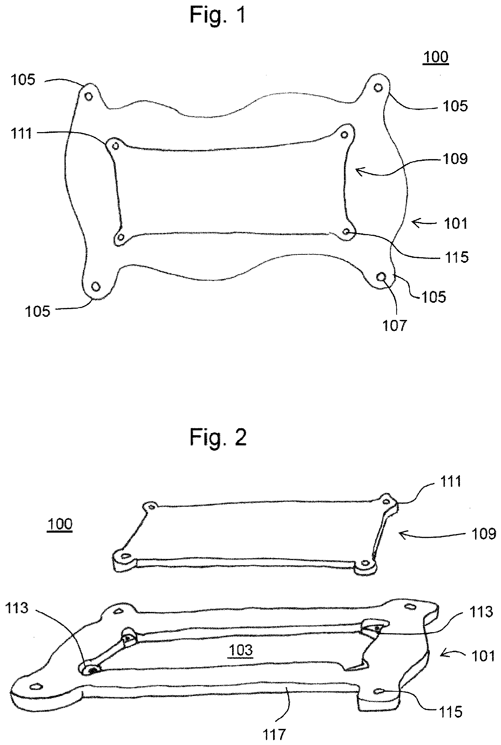

Figures 1 to 3 show various representations of a cranial prosthesis 100 embodying a number of different aspects of the present invention.

The cranial prosthesis comprises a single peripheral frame portion 101 defining a single central aperture 103. In other words, the prosthesis is a two-part prosthesis. The peripheral frame portion here comprises the entire periphery of the prosthesis 100. The frame portion is an irregular shape selected to match a cranial defect of an intended recipient of the prosthesis, and accordingly improve cosmesis of a cranioplasty using the implant. The frame portion has four integrated fixation lugs 105 which are approximately evenly distributed about the perimeter of the peripheral frame portion 101. Each lug has a hole 107 for receiving a screw (not shown) for fixing the lug to the skull of a recipient of the prosthesis in a manner well known to the skilled person. Providing integrated fixation lugs gives improved intraoperative ease, as it removes the requirement for provision and attachment of separate fixation plates: the prosthesis can be attached directly to the skull of the recipient.

The prosthesis comprises a removable insert portion 109 which is of a shape suitable for closing the aperture 103. Here, the removable insert portion entirely closes the aperture 103 when it is located within the aperture, such as after implantation of the prosthesis. The removable insert portion also has four integrated fixation lugs 111 for removably attaching the insert portion 109 to the peripheral frame portion 101. The integrated fixation lugs 111 and the shape of the peripheral frame portion 101 are selected to provide an appropriate degree of keying between the lugs and the frame which can assist in correct location of the insert within the aperture. In a similar manner to the lugs 105 of the peripheral frame portion, each fixation lug 111 of the insert portion has a hole 115 receiving a screw (not shown) for fixing the lug to the peripheral frame portion. Here, the (countersunk) holes 107, 115 have chamfered edges to allow for recessing of a screw head into the hole. The holes 107, 115 could alternatively be counterbored.

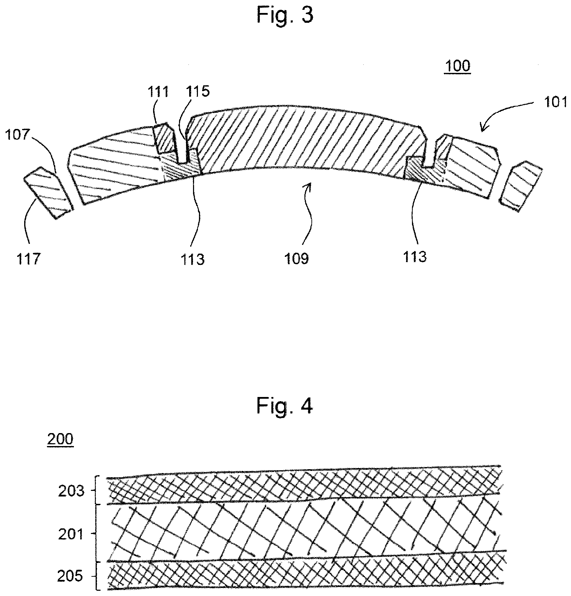

The fixation lugs 111 of the insert portion attach to the peripheral frame potion 101 at recessed fixation points comprising suitably shaped ledges 113 by which the fixation lugs of the insert portion can be supported. Because the ledges 113 are recessed from the surface of the peripheral frame portion, the outer surface of the insert lies flush with the outer surface of the peripheral frame portion when it is closing the aperture 103. This can be seen most clearly in Fig. 3.

The peripheral frame portion and the insert portion have different porosities represented by the different density of hashing in Fig. 3. Here, the peripheral frame portion has a higher porosity than the insert portion. By providing a prosthesis where the porosity is graduated laterally across the implant in an in-plane direction, the prosthesis is able to provide suitable bone integration at the peripheral frame portion (which has a bone-contacting edge 117) whilst also providing a satisfactory mechanical strength. Furthermore, bone integration into the insert portion is discouraged due to its lower porosity. The recessed fixation ledges 113 of the peripheral frame portion have a lower porosity than the rest of the peripheral frame portion. This allows for the ledges to have suitable mechanical strength for holding a screw in place.

The cranial prosthesis shown in Fig. 1-3 is advantageously made from a material according to Example 1, below. That is, the prosthesis is formed from 29.5 -34.5 wt% PPSU reinforced composite containing 65 - 70wt% HAP nanoparticle and 0.5wt% reduced graphene oxide/titanium dioxide nanocomposite based on total weight of the composite material, the reduced graphene oxide/titanium dioxide nanocomposite having a TiO₂:rGO ratio of 80:20 by weight. In this way, the prosthesis can provide suitable promotion of osseointegration in combination with antibacterial capability.

Figure 4 is a schematic cross-sectional view of a portion of a cranial prosthesis 200 according to one embodiment of the present invention. The cranial prosthesis 200 has a core layer 201, a first skin layer 203 and a second skin layer 205. The skin layers are disposed on opposite sides of the core layer 201 in a symmetrical arrangement. The different porosities of the different layers of the prosthesis (as represented by the density of hashing) provide a through-thickness porosity graduation. The skin layers have a lower porosity than the core layer, thus more closely matching or mimicking the structure and morphology of cranial bone, in which the central trabecular bone layer has greater porosity than the surrounding layers of cortical bone.

Here, the core layer is thicker than each of the skin layers, being approximately twice as thick as each skin layer. The skin layers are of approximately equal thickness. This arrangement also mimics that of cranial bone, where the central trabecula bone layer is typically thicker than the cortical bone layers.

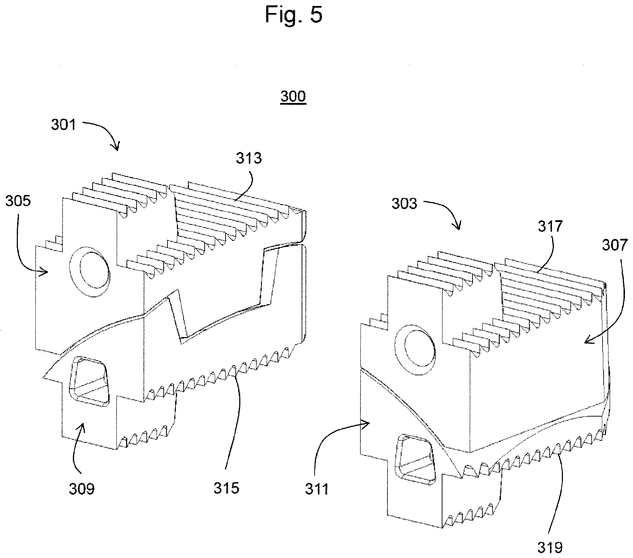

Figures 5 to 7 show various oblique views of a spinal prosthesis 300 and members forming parts of the spinal prosthesis as disclosed and described in WO 2006/114646. The prosthesis is an articulating intervertebral disc prosthesis including a first body 301 and a second body 303. Each of the first and second bodies comprises a respective first member 305, 307 and a second member 309, 311. The first body first member 305 has a first vertebra contacting portion 313, and the first body second member 309 has a second vertebra contacting portion 315. Likewise, the second body first member 307 has a first vertebra contacting portion 317, and the second body second member portion 311 has a second vertebra contacting portion 319. Here, the vertebra contacting portions 313, 315, 317, 319 are profiled to be a buttress-thread type ridged surface to prevent translation of the prosthesis when the prosthesis is in place in the spine and in use.

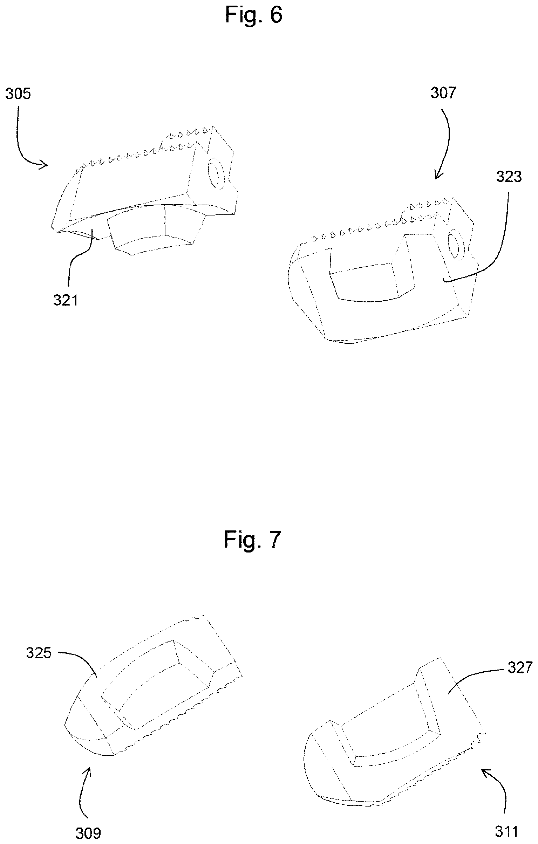

Each of the first members 305, 307 has a bearing surface 321, 323 (respectively) as shown in Fig. 6. Each of the second members 309, 311 has a bearing surface 325, 327 (respectively) as shown in Fig. 7. Here, each bearing surface 321, 323, 325, 327 is formed to approximate to a portion of an oblate spheroidal surface. In use, the first and second body first member bearing surfaces 321, 323 abut the respective second member bearing surfaces 325, 327.

Because the prosthesis is an articulating prosthesis having multiple bearing surfaces, it is advantageously made from a material according to Example 2, below. That is, the prosthesis is formed from 98- 99 wt% PPSU reinforced composite containing 1-2wt% reduced graphene oxide/titanium dioxide nanocomposite based on total weight of the composite material, the reduced graphene oxide/titanium dioxide nanocomposite having a TiO₂:rGO ratio of 80:20 by weight. In this way, the prosthesis can provide improved tribological performance in combination with antibacterial capability.

### Porosity Measurement

Porosity as discussed in the above description refers to bulk porosity defined as 1-Vₛ, where Vₛ = solid volume fraction of the material. The solid volume fraction for a sample can be calculated by calculating the ratio between the density of the sample and the theoretical density of a non-porous sample of the same material. However, the skilled person will be well aware of a wide range of suitable methods for measuring bulk porosity of a material.

Where the above description contains detail of porosities and pore sizes of natural bone, these estimates are calculated from methods including e.g. magnetic resonance imaging (MRI), micro computer tomography (mCT) or microradiography.

Whilst the above description generally refers to bulk porosity, the skilled person will also understand that it is possible to calculate the interconnected porosity or pore volume of a material using e.g. mercury intrusion porosimetry (MIP). The materials and devices disclosed herein preferably have an interconnected porosity/pore volume which promotes osseointegration.

### Pore size measurement

Pore size as discussed in the above description refers to average diameter of pores within a selected region. The skilled person will be well aware of a wide range of suitable methods for measuring average pore diameter, including e.g. optical microscopy (OM), scanning electron microscopy (SEM), X-Ray scanning, and CT scanning.

### Examples

### EXAMPLE MATERIAL 1

One composite material according to the present invention is as follows:
29.5 -34.5 wt% PPSU reinforced composite containing 65 -70wt% hydroxyapatite (HAP) nanoparticle and 0.5wt% reduced graphene oxide/titanium dioxide nanocomposite based on total weight of the composite material, the reduced graphene oxide/titanium dioxide nanocomposite having a TiO₂:rGO ratio of 80:20 by weight.

Such material may be particularly suitable for promoting osseointegration in combination with antibacterial capability.

### EXAMPLE MATERIAL 2

Another composite material according to the present invention is as follows:
98- 99 wt% PPSU reinforced composite containing 1-2wt% reduced graphene oxide/titanium dioxide nanocomposite based on total weight of the composite material, the reduced graphene oxide/titanium dioxide nanocomposite having a TiO₂:rGO ratio of 80:20 by weight.

Such material may be particularly suitable for providing enhanced tribological performance in combination with antibacterial capability.

### EXAMPLE MATERIAL SPECIFICATIONS

The initial materials used in manufacture of the above composite materials above are standard, commercially-available materials such as the following:
PPSU: Solvay Specialty Polymers VERIVA^{™} VR-500 Polyphenylsulfone having the following typical properties:

**Table 1: VERIVA^{™} VR-500 material specification**

| **Property** | **Value** | **Test Method** |
|---|---|---|
| Specific Gravity | 1.27 to 1.31 g/cc | ASTM D792 |
| Melt Mass-Flow Rate (MFR) (365°C/5.0 kg) | 13 to 20 g/10 min | ASTM D1238 |
| Tensile Modulus | > 275000 psi | ASTM D638 |
| Tensile Strength (Yield) | > 10000 psi | ASTM D638 |
| Tensile Elongation (Break) | > 6.5 % | ASTM D638 |
| Notched Izod Impact | > 10 ft·lb/in | ASTM D256 |
| Cytotoxocity | Pass | ISO 10993:5 |
| Physiochemical Testing | Pass | ISO 10993:18 |

TiO2: 99.9% purity, anastase phase, 18nm (US Research Nanomaterials) having the following typical properties:

**Table 2: 99.9% purity pure anastase 18nm (US Research Nanomaterials) material specification**

| **Property** | **Value** |
|---|---|
| Purity | 99.9% |
| Average particle diameter | 18 nm |
| Specific surface area | 200-240 m²/g |
| Color | white |
| Bulk Density | 0.24 g/cm³ |
| True Density | 3.9 g/cm³ |
| PH | 6-6.5 |

Graphene oxide: Research Grade Single Layer Graphene Oxide Dry Nanopowder (US Research Nanomaterials) having the following typical properties:

**Table 3: Research Grade Single Layer Graphene Oxide Dry Nanopowder (US Research Nanomaterials) material specification**

| **Property** | **Value** |
|---|---|
| Purity | >99.3% Single layer graphene Oxide |
| Trace elements | C: 68.44%, O: 30.92, Free C: 0.4%, S: 0.13% |
| Diameter | 1.5-5.5 µm |
| Thickness | 0.43-1.23 nm |
| Colour | Amber |

Hydroxyapatite: nanoXIM HAp200 (Fluidinova) having the following typical properties:

**Table 4: nanoXIM HAp200 (Fluidinova) material specification**

| **Property** | **Value** |
|---|---|
| Phase purity, Ca₁₀(PO₄)₆(OH)₂ | 100% |
| Specific surface area BET, | ≥ 100 m2/g |
| Heavy metals, as Pb, | ≤ 20 ppm |
| Particle size, d₅₀ | 5.0±1 µm, 10.0±2 µm |

The composite materials of Example 1 and Example 2 may be made from these initial materials using any suitable method known in the state of the art, including but not limited to acoustic mixing, acoustic milling ultrasonification, mechanical paddle mixing, melt flow mixing, static melt blending or spiral flow jet milling to disperse or distribute the filler material components throughout the polymeric matrix material in a known manner. The filler material or filler material components may initially be provided as a suspension or dispersion in a suitable aqueous or polar solvent e.g. ethanol.

In some cases it may be advantageous to first dissolve the polymeric matrix material in a suitable solvent before addition of the filler material, however this may not be necessary where the polymeric matrix material is hot melt processable e.g. in powder, or granule form.

In some cases it may be preferred to first form a TiO₂-Graphene nanocomposite from the initial materials before combining this nanocomposite filler material with the matrix material. One method for preparation of TiOz-reduced graphene oxide (rGO) nanocomposites using UV-assisted photocatalytic reduction is described in a paper by Williams et al.: "TiOz-Graphene Nanocomposites. UV-Assisted Photocatalytic Reduction of Graphene Oxide," ACS Nano, (2008), 2 (7), 1487-1491. Another method is described in Yang et al. "Synthesis of r-GO/TiO2composites via the UV-assisted photocatalytic reduction of graphene oxide" (2016). In this method, irradiation is performed for 5 hours using a low pressure mercury lamp having a narrow band UV emission at a wavelength of 254nm and a power of 18W.

An alternative method for preparation of TiO₂-reduced graphene oxide (rGO) nanocomposites is via a hydrothermal method, such as that described in a paper by Fan et al.: "Nanocomposites of TiO2 and Reduced Graphene Oxide as Efficient Photocatalysts for Hydrogen Evolution": J. Phys. Chem. C 2011, 115, 10694-10701.

The photoactive nanocomposite may then be dispersed through the polymeric matrix material using one of the methods discussed above.

Photoactivation (or top-up photoactivation) of the nanocomposite material may be performed, before or after dispersion of the nanocomposite in the polymeric matrix material.

The resulting powder/polymer aggregate may then be pelletised, extruded, compression or injection moulded, thermoformed or 3D printed. E.g., the powder/polymer aggregate may be extruded via a hot melt process to form filament for use in fusion deposition modelling.

### EXAMPLE METHOD FOR PRODUCTION OF COMPOSITE MATERIAL FILAMENT

One suitable method for production of a TiO₂ /rGO PPSU nanocomposite filament having a composition of e.g. Example Material 1 is as follows. The quantities of each material are not specified below as this method may be used to manufacture TiO₂/rGO PPSU nanocomposite filaments having a range of compositions. Accordingly, the quantities of each material may be selected as appropriate for the desired composition of the composite material.
A. Ultrasonication of a graphene oxide (GO) dispersion in water or ethanol for 60mins whilst adding TiO₂ to form a colloidal suspension of TiO₂ and GO.
B. Transfer of said suspension to a suitable immersion well photoreactor, where the suspension undergoes UV-assisted photocatalytic reduction for at least 5 hrs but more preferably 24-48hrs under nitrogen purge & continuous stirring to maximise yield of photoactivated reduced graphene oxide/titanium dioxide nanocomposite powder (rGO)/TiO2)
C. Separation of the TiO₂/rGO nanocomposite powder by centrifuging at 5000rpm for 30mins.
D. Washing and filtration of the separated TiO₂/rGO nanocomposite powder (washing and filtration may be performed multiple times), followed by vacuum drying for 4 hrs at 60 °C
E. Addition of the dried powder to a coarse mixture of pre-dried HAP nanopowder and PPSU granules or powder.
F. Acoustic mixing/milling of the TiO₂/rGO/HAP/PPSU mixture for 1-2hrs to produce the final polymer/powder aggregate.
G. Vacuum drying of the powder/polymer aggregate for 4 hrs at 60 °C prior to hot-melt flow mixing and/or static melt blending and hot melt extrusion to form filament.
H. Air drying of the filament at 80 °C for 12 hrs prior to FDM printing.

### FUSION DEPOSITION MODELLING

As mentioned above, materials and devices according to the present invention may be processed or manufactured using fusion deposition modelling techniques. The skilled person will be aware of commercially available fusion deposition modelling machines having resolutions as low as 50 µm or less, which would be suitable for use in processing and/or manufacturing materials and devices as discussed above.

To generate a prosthesis by fusion deposition modelling, an STL or OBJ file of the desired product should first be generated (e.g. for a cranial plate by converting patient specific high resolution 3D CT, MRI or surface scan data to an STL or OBJ file using software in a manner well known to the skilled person). The model can then be imported into software associated with the FDM machine, which computes the required tool paths for the extrusion nozzle of the FDM machine. The extrusion nozzle then draws cross-sectional layers one at a time in the X, Y, Z coordinates using a heated material extrusion process to form the FDM printed prosthesis.

Example FDM processing parameters for production of an orthopaedic, spinal or craniomaxillofacial implant from the material of the first aspect including PPSU as the polymeric matrix material are as follows:
A) Filament air drying prior to printing at about 80°C for 12hrs
B) Extruder nozzle diameter: 0.2mm
   Layer height: 0.05 - 0.1mm
   Print speed: 10 - 80 mm/s (preferably around 10 mm/s)
C) Printer extruder nozzle temperature:
   260-400 °C
   Heated build platform: ≥ 150 °C
   Heated build chamber: ≥ 70 °C
D) Post-print surface finishing followed by annealing

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" or "around" one particular value, and/or to "about" or "around" another particular value. When such a range is expressed, another embodiment includes from the one particular value and/or to the other particular value. Similarly, when values are expressed as approximations, by the use of the antecedents "about" or "around", it will be understood that the particular value forms another embodiment. The term "about" in relation to a numerical value is optional and means for example +/-10%.

### References

A number of publications are cited above in order to more fully describe and disclose the invention and the state of the art to which the invention pertains. Full citations for these references are provided below.
The Williams' Dictionary of Biomaterials, D.F. Williams, Liverpool University Press, 1999
Visai et al.: Titanium oxide antibacterial surfaces in biomedical devices; Int J Artif Organs (2011); 34 (9); 929-946
Diez-Pascual et al.: Effect of TiO2 nanoparticles on the performance of polyphenylsulfone biomaterial for orthopaedic implants; J Materials Chemistry B; (2014); 2; 7502-7514
Williams et al.: TiO2-Graphene Nanocomposites. UV-Assisted Photocatalytic Reduction of Graphene Oxide; ACS Nano, (2008), 2 (7), 1487-1491
Kralchevska et al: Influence of gamma-irradiation on the photocatalytic activity of Degussa P25 TiO2; J Mater Sci; (2012); 47; 4936-4945
Fan et al.: Nanocomposites of TiO2 and Reduced Graphene Oxide as Efficient Photocatalysts for Hydrogen Evolution': J. Phys. Chem. C 2011, 115, 10694-10701
Yang et al : 'Synthesis of r-GO/TiO2composites via the UV-assisted photocatalytic reduction of graphene oxide': Applied Surface Science 380 (2016) 249-256

## Claims

1. An orthopaedic implant comprising a composite material, the composite material comprising:
a polymeric matrix material; and
filler material comprising a TiO₂-reduced graphene oxide nanocomposite.

2. An orthopaedic implant according to claim 1 wherein the polymeric matrix material is selected from polyphenylsulphone, polycarbonate urethane, a PAEK (polyaryletherketone) polymer, polycaprolactone, poly -L- lactic acid, polyvinyl acetate or poly(lactic -co-glycolic acid).

3. An orthopaedic implant according to any one of the preceding claims wherein the amount of filler material is from 0.05 wt% to 80 wt% based on total weight of the composite material.

4. An orthopaedic implant according to any one of the preceding claims wherein the amount of TiO₂ is less than 3wt% based on total weight of the composite material.

5. An orthopaedic implant according to any one of the preceding claims wherein the combined amount of TiO₂ and reduced graphene oxide is less than 3 wt% based on total weight of the composite material.

6. An orthopaedic implant according to any one of the preceding claims wherein the ratio of TiO₂ to reduced graphene oxide in the filler material is between 50:50 and 99:1 by weight.

7. An orthopaedic implant according to claim 6 wherein the ratio of TiO₂ to reduced graphene oxide in the filler material is 80:20 by weight.

8. An orthopaedic implant according to any one of the preceding claims wherein the filler material further comprises hydroxyapatite.

9. An orthopaedic implant according to claim 8 wherein the amount of hydroxyapatite is from 1 wt% to 70 wt% based on total weight of the composite material.

10. An orthopaedic implant according to any one of the preceding claims wherein the TiO₂-reduced graphene oxide nanocomposite is in a photoactivated state.

11. An orthopaedic implant according to claim 10 wherein the photoactivated state is achieved by irradiation of the TiO₂-reduced graphene oxide nanocomposite, optionally with ultraviolet or gamma radiation.

12. An orthopaedic implant according to claim 11 wherein the radiation is gamma radiation applied at a dose of 8-40 kGy, optionally at a dose of 25 kGy.

13. An orthopaedic implant according to any one of the preceding claims wherein the orthopaedic implant is a cranial prosthesis.

14. An orthopaedic implant according to any one of the preceding claims wherein the orthopaedic implant is an articulating implant having one or more bearing surfaces.

15. An orthopaedic implant according to claim 13 or claim 14 wherein the device is an orthopaedic implant having variable porosity.

## Patentansprüche

1. Orthopädisches Implantat, das ein Verbundmaterial umfasst, wobei das Verbundmaterial Folgendes umfasst:
ein Polymermatrixmaterial; und
einen Füllstoff, der eine TiO₂-reduzierte Graphenoxid-Nanoverbundstruktur umfasst.

2. Orthopädisches Implantat nach Anspruch 1, wobei das Polymermatrixmaterial aus Polyphenylsulfon, Polycarbonatturethan, einem PAEK- (Polyaryletherketon-) Polymer, Polycaprolacton, Poly-L-milchsäure, Polyvinylacetat und Poly(lactid-co-glycolid) ausgewählt ist.

3. Orthopädisches Implantat nach einem der vorangegangenen Ansprüche, wobei die Menge des Füllstoffs 0,05 Gew.-% bis 80 Gew.-%, bezogen auf das Gesamtgewicht des Verbundmaterials beträgt.

4. Orthopädisches Implantat nach einem der vorangegangenen Ansprüche, wobei die Menge an TiO₂ weniger als 3 Gew.-%, bezogen auf das Gesamtgewicht des Verbundmaterials beträgt.

5. Orthopädisches Implantat nach einem der vorangegangenen Ansprüche, wobei die kombinierte Menge von TiO₂ und reduziertem Graphenoxid weniger als 3 Gew.-%, bezogen auf das Gesamtgewicht des Verbundmaterials beträgt.

6. Orthopädisches Implantat nach einem der vorangegangenen Ansprüche, wobei das Gewichtsverhältnis zwischen TiO₂ und dem reduzierten Graphenoxid im Füllstoff zwischen 50:50 und 99:1 liegt.

7. Orthopädisches Implantat nach Anspruch 6, wobei das Gewichtsverhältnis zwischen TiO₂ und dem reduzierten Graphenoxid im Füllstoff 80:20 beträgt.

8. Orthopädisches Implantat nach einem der vorangegangenen Ansprüche, wobei der Füllstoff weiters Hydroxylapatit umfasst.

9. Orthopädisches Implantat nach Anspruch 8, wobei die Menge an Hydroxylapatit 1 Gew.-% bis 70 Gew.-%, bezogen auf das Gesamtgewicht des Verbundmaterials beträgt.

10. Orthopädisches Implantat nach einem der vorangegangenen Ansprüche, wobei die TiO₂-reduzierte Graphenoxid-Nanoverbundstruktur in einem photoaktivierten Zustand vorliegt.

11. Orthopädisches Implantat nach Anspruch 10, wobei der photoaktivierte Zustand durch Bestrahlung der TiO₂-reduzierten Graphenoxid-Nanoverbundstruktur, gegebenenfalls mit UV- oder Gamma-Strahlung, erreicht wird.

12. Orthopädisches Implantat nach Anspruch 11, wobei die Strahlung Gamma-Strahlung ist, die in einer Dosis von 8-40 kGy, gegebenenfalls in einer Dosis von 25 kGy, angewandt wird.

13. Orthopädisches Implantat nach einem der vorangegangenen Ansprüche, wobei das orthopädische Implantat eine Schädelprothese ist.

14. Orthopädisches Implantat nach einem der vorangegangenen Ansprüche, wobei das orthopädische Implantat ein gelenkiges Implantat mit einer oder mehreren Auflageflächen ist.

15. Orthopädisches Implantat nach Anspruch 13 oder 14, wobei die Vorrichtung ein orthopädisches Implantat mit variabler Porosität ist.

## Revendications

1. Implant orthopédique comprenant un matériau composite, le matériau composite comprenant :
un matériau de matrice polymère ; et
un matériau de charge comprenant un nanocomposite d'oxyde de graphène réduit par TiO₂.

2. Implant orthopédique selon la revendication 1, dans lequel le matériau de matrice polymère est choisi parmi la polyphénylsulfone, le polycarbonate-uréthane, un polymère de PAEK (polyaryléthercétone), la polycaprolactone, l'acide poly-L-lactique, l'acétate de polyvinyle ou l'acide poly(lactique-co-glycolique).

3. Implant orthopédique selon l'une quelconque des revendications précédentes, dans lequel la quantité de matériau de charge est de 0,05 % en poids à 80 % en poids sur la base du poids total du matériau composite.

4. Implant orthopédique selon l'une quelconque des revendications précédentes, dans lequel la quantité de TiO₂ est inférieure à 3 % en poids sur la base du poids total du matériau composite.

5. Implant orthopédique selon l'une quelconque des revendications précédentes, dans lequel la quantité combinée de TiO₂ et d'oxyde de graphène réduit est inférieure à 3 % en poids sur la base du poids total du matériau composite.

6. Implant orthopédique selon l'une quelconque des revendications précédentes, dans lequel le rapport du TiO₂ à l'oxyde de graphène réduit dans le matériau de charge est compris entre 50:50 et 99:1 en poids.

7. Implant orthopédique selon la revendication 6, dans lequel le rapport du TiO₂ à l'oxyde de graphène réduit dans le matériau de charge est de 80:20 en poids.

8. Implant orthopédique selon l'une quelconque des revendications précédentes, dans lequel le matériau de charge comprend en outre de l'hydroxyapatite.

9. Implant orthopédique selon la revendication 8, dans lequel la quantité d'hydroxyapatite est de 1 % en poids à 70 % en poids sur la base du poids total du matériau composite.

10. Implant orthopédique selon l'une quelconque des revendications précédentes, dans lequel le nanocomposite de TiO₂-oxyde de graphène réduit est dans un état photoactivé.

11. Implant orthopédique selon la revendication 10, dans lequel l'état photoactivé est obtenu par irradiation du nanocomposite de TiO₂-oxyde de graphène réduit, facultativement avec un rayonnement ultraviolet ou gamma.

12. Implant orthopédique selon la revendication 11, dans lequel le rayonnement est un rayonnement gamma appliqué à une dose de 8 à 40 kGy, facultativement à une dose de 25 kGy.

13. Implant orthopédique selon l'une quelconque des revendications précédentes dans lequel l'implant orthopédique est une prothèse crânienne.

14. Implant orthopédique selon l'une quelconque des revendications précédentes, dans lequel l'implant orthopédique est un implant d'articulation présentant une ou plusieurs surfaces d'appui.

15. Implant orthopédique selon la revendication 13 ou la revendication 14, dans lequel le dispositif est un implant orthopédique présentant une porosité variable.
